(19) Europäisches Patentamt
European Patent Office
Office européen des brevets

(11) **EP 3 748 536 B1**

(12) **EUROPÄISCHE PATENTSCHRIFT**

(45) Veröffentlichungstag und Bekanntmachung des Hinweises auf die Patenterteilung:
**01.10.2025 Patentblatt 2025/40**

(21) Anmeldenummer: **20176452.9**

(22) Anmeldetag: **26.05.2020**

(51) Internationale Patentklassifikation (IPC):
*A61B 5/11* (2006.01)     *G16H 40/20* (2018.01)
*G06V 20/52* (2022.01)     *G06V 40/20* (2022.01)
*G08B 13/196* (2006.01)

(52) Gemeinsame Patentklassifikation (CPC):
**A61B 5/1128; A61B 5/1113; G06V 20/52; G06V 40/20; G08B 13/19613; G16H 40/20**

(54) **SYSTEM UND VERFAHREN ZUR AUTOMATISCHEN ERKENNUNG VON GEFÄHRDUNGSSITUATIONEN**

METHOD AND SYSTEM FOR AUTOMATICALLY DETECTING DANGEROUS SITUATIONS

SYSTÈME ET PROCÉDÉ DE DÉTECTION AUTOMATIQUE DE SITUATIONS DANGEREUSES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.06.2019 DE 102019003999**

(43) Veröffentlichungstag der Anmeldung:
**09.12.2020 Patentblatt 2020/50**

(73) Patentinhaber: **Drägerwerk AG & Co. KGaA 23558 Lübeck (DE)**

(72) Erfinder:
• **Franz, Frank**
  **23558 Lübeck (DE)**
• **Schlichting, Stefan**
  **23558 Lübeck (DE)**
• **Diesel, Jasper**
  **23558 Lübeck (DE)**

(56) Entgegenhaltungen:
WO-A1-2019/011769     CN-A- 106 233 349
US-A1- 2019 340 441     US-B1- 10 043 360

EP 3 748 536 B1

**Beschreibung**

Die Erfindung betrifft ein System und ein Verfahren zur automatischen Erkennung von Gefährdungssituationen innerhalb eines Überwachungsbereichs für Objekte im medizinischen Umfeld. Weiterhin betrifft die Erfindung ein Programm mit einem Programmcode zur Durchführung des erfindungsgemäßen Verfahrens.

Im medizinischen Umfeld ist es ein bekanntes Problem, dass verwirrte Patienten ihre eigene Behandlung gefährden, indem sie sich beispielsweise Verbände entfernen, Katheter herausziehen oder selbst mit komplizierten medizinischen Geräten interagieren. Um derartige Aktivitäten des Patienten in dessen eigenem Interesse rechtzeitig zu unterbinden, ist bekannt, ein kamerabasiertes Überwachungssystem zur Überwachung von Bettplätzen innerhalb eines Krankenhauses vorzusehen.

US 10,090,068 beschreibt ein Verfahren, durch das im klinischen Umfeld überwacht wird, ob eine Hand eines Patienten für eine vorbestimmte Mindestdauer in eine vorbestimmte Zone innerhalb eines Überwachungsbereiches eindringt. Die vorbestimmte Zone kann beispielsweise im Bereich eines Katheters oder im Bereich einer Wunde des Patienten liegen. Dadurch ermöglicht das beschriebene Verfahren ein Eindringen der Hand des Patienten in einen für diesen gefährlichen Bereich zu erkennen.

CN 106 233 349 A beschreibt ein Verfahren zur Überwachung eines Patienten innerhalb eines medizinischen Überwachungsbereichs mittels eines eine 3D-Kameraeinrichtung aufweisenden Überwachungssystems, mit den folgenden Schritten: Erzeugen einer Punktwolke des Überwachungsbereichs mittels des Überwachungssystems, Auswerten der Punktwolke zum Erkennen von vordefinierten Objekten, insbesondere von Personen, Ermitteln des Ortes mindestens eines erfassten Objekts im Überwachungsbereich, Vergleichen des ermittelten Ortes des mindestens einen erfassten Objekts mit mindestens einem vordefinierten Wert für den Ort des erfassten Objekts.

US 10 043 360 B1 beschreibt ein System zur Abschreckung von organisierter Einzelhandelskriminalität.

WO 2019 / 011 769 A1 beschreibt ein Verfahren, eine Vorrichtung und ein Computerprogramm zur Erfassung von optischen Bilddaten einer Patientenumgebung und zur Erkennung einer Patientenüberprüfung. Das Verfahren umfasst ein Detektieren eines Patienten basierend auf den Bilddaten, ein Detektieren zumindest einer weiteren Person basierend auf den Bilddaten und ein Bestimmen zumindest einer geometrischen Relation zwischen dem Patienten und der zumindest einen weiteren Person, wobei das Bestimmen der geometrischen Relation ein Bestimmen einer Orientierung oder einer Blickrichtung der zumindest einen weiteren Person in Relation zu dem Patienten umfasst. Das Verfahren umfasst ferner ein Erkennen der Patientenüberprüfung basierend auf der geometrischen Relation.

Aufgabe der vorliegenden Erfindung ist es, eine verbesserte automatisierte Erkennung von Gefährdungssituationen für Objekte im medizinischen Umfeld, insbesondere ein verbessertes Vermeiden der Ausgabe von Fehlalarmen und insbesondere ein frühes Erkennen von relevanten Gefährdungssituationen, zu ermöglichen.

Erfindungsgemäß wird zur Lösung dieser Aufgabe ein System zur automatischen Erkennung von Gefährdungssituationen innerhalb eines Überwachungsbereichs für Objekte im medizinischen Umfeld vorgeschlagen, mit einer Sensoreinheit, einer Prozessoreinheit und einer Ausgabeeinheit.

Die Sensoreinheit weist eine Anzahl von optischen Sensoren auf und ist ausgebildet, ein Sensorsignal zu bestimmen und in Echtzeit auszugeben. Dabei ist die Anzahl von optischen Sensoren weiterhin derart in dem medizinischen Umfeld anordenbar und derart ausgebildet, dass das Sensorsignal Sensordaten umfasst, die eine Abfolge von dreidimensionalen Darstellungen des Überwachungsbereichs über eine Überwachungsdauer indizieren.

Die Prozessoreinheit ist ausgebildet, das Sensorsignal zu empfangen und anhand der indizierten dreidimensionalen Darstellungen eine Festlegung einer Anzahl zu schützender Objekte innerhalb des Überwachungsbereichs auszuführen. Weiterhin ist die Prozessoreinheit ausgebildet, anhand der indizierten dreidimensionalen Darstellungen eine Erkennung, insbesondere eine automatisierte Erkennung, von gefährdenden Objekten innerhalb des Überwachungsbereichs auszuführen und eine Anzahl von Objektpaaren durch eine Zuordnung zwischen einem zu schützenden Objekt aus der Anzahl zu schützender Objekte und einem gefährdenden Objekt zu bestimmen. Weiterhin ist die Prozessoreinheit ausgebildet, im Laufe der Überwachungsdauer die Positionen von zu schützendem Objekt und gefährdendem Objekt eines jeweiligen Objektpaares innerhalb des Überwachungsbereichs zu überwachen und abhängig von den beiden aktuellen Positionen und einer Gruppe von sich aus einer Auswertung früherer Auswertedaten ergebender Parameter einem jeweiligen Objektpaar einen aktuellen Gefährdungswert zuzuordnen, wobei die sich ergebenden Parameter zumindest eine Distanz zwischen den Objekten eines Objektpaares und eine Richtung einer Bewegung der Objekte eines Objektpaares relativ zueinander sind. Ferner ist die Prozessoreinheit weiterhin ausgebildet, über ein zeitliches Auswertungsintervall, insbesondere über ein vorbestimmtes zeitliches Auswertungsintervall, für jedes Objektpaar jeweils die aktuellen Gefährdungswerte bis zum aktuellen Zeitpunkt zu verrechnen und ein entsprechendes Alarmierungssignal auszugeben, falls ein so verrechneter Gefährdungsbetrag eines Objektpaares einen vorbestimmten Schwellenwert übersteigt.

Die Ausgabeeinheit ist ausgebildet, das Alarmierungssignal zu empfangen und abhängig von dem Alarmierungssignal einen Alarm auszulösen.

Im Rahmen der Erfindung wurde erkannt, dass zur Vermeidung von Fehlalarmen und für eine besonders frühe Erkennung von Gefahren eine aktuelle Situation innerhalb des Überwachungsbereichs qualitativ ausgewertet werden muss.

Hierfür werden erfindungsgemäß zu schützende Objekte festgelegt und gefährdende Objekte innerhalb des Überwachungsbereichs erkannt. Weiterhin berücksichtigt das erfindungsgemäße System für eine derartige qualitative Auswertung neben aktuellen Daten auch sich aus früheren Auswertedaten ergebende Parameter.

Als zu vermeidender Gefahrenfall wird im Rahmen der Erfindung eine Berührung von zu schützenden Objekt und gefährdendem Objekt angesehen, da eine derartige Berührung im medizinischen Alltag nachteilige Folgen für die Patientengesundheit haben kann, beispielsweise durch eine Fehlbedienung eines medizinischen Gerätes oder durch ein Herausreißen eines Tubus oder eines Katheters durch den Patienten.

Durch das erfindungsgemäße System wird vorteilhaft sichergestellt, dass Gefährdungssituationen innerhalb des Überwachungsbereichs schnell erkannt werden können. Insbesondere muss nicht abgewartet werden, bis eine gefährliche Situation entsteht, beispielsweise durch ein Einführen einer Patientenhand in ein gefährdetes Gebiet, beispielsweise in die Nähe eines Katheters. So wird frühzeitig durch die Überwachung der beiden aktuellen Positionen in Kombination mit der Auswertung früherer Auswertedaten ein besonders aussagekräftiger Gefährdungswert ermittelt, aus dem sich der die aktuelle Gefahr charakterisierende Gefährdungsbetrag eines jeweiligen Objektpaares ergibt.

Durch die Verwendung eines vorbestimmten Schwellenwerts kann vorteilhaft benutzerspezifisch vorgegeben werden, ab welcher Wahrscheinlichkeit, dass eine Gefährdung vorliegen könnte, ein entsprechendes Alarmierungssignal ausgegeben wird. Falls der Schwellenwert derart vorbestimmt wird, dass bereits bei einer geringen Wahrscheinlichkeit einer Gefährdung das Alarmierungssignal ausgegeben wird, würde dies wiederum zu einer höheren Anzahl an Fehlalarmen führen, als wenn erst bei einer moderaten Wahrscheinlichkeit einer Gefährdung das Alarmierungssignal ausgegeben wird.

Die Festlegung der Anzahl zu schützender Objekte kann beispielsweise automatisiert, manuell oder durch Gesten-Steuerung erfolgen.

Die Erkennung von gefährdenden Objekten erfolgt besonders bevorzugt automatisiert. Eine derartige automatisierte Erkennung erlaubt vorteilhaft eine schnelle Erkennung dieser Objekte, da nicht erst eine manuelle Eingabe abgewartet werden muss. Weiterhin ermöglicht dies eine Erkennung von gefährdenden Objekten, denen ein Nutzer des Systems möglicherweise gar kein Gefährdungspotenzial zugesprochen hätte. Diese automatisierte Erkennung erfolgt vorzugsweise durch das Ausführen eines Algorithmus zur Objekterkennung, durch den der Überwachungsbereich nach vordefinierten Objekten abgesucht wird. Derartige Algorithmen sind wohlbekannt und werden daher im Folgenden nicht detailliert erläutert. Die automatisierte Erkennung kann auch aufgrund von Objekteigenschaften, wie beispielsweise einer erfassten Objektgeschwindigkeit oder einer Bewegungsrichtung eines Objekts erfolgen. Insbesondere kann die automatisierte Erkennung auf einer Auswertung der Abfolge von dreidimensionalen Daten basieren. Alternativ oder ergänzend können die gefährdenden Objekte manuell oder durch Gesten-Steuerung erkannt und dadurch für die Prozessoreinheit festgelegt werden.

Die Sensordaten können erfindungsgemäß eine zweidimensionale Anordnung indizierende Daten sein, die es erlauben, eine dreidimensionale Darstellung des Überwachungsbereiches zu bestimmen. Die erfindungsgemäße Abfolge von dreidimensionalen Darstellungen ergibt sich aus einer zeitlichen Abfolge von einzelnen Sensordaten zu dem in Echtzeit ausgegebenen Sensorsignal. Dreidimensionale Darstellungen können erfindungsgemäß auch dreidimensionale Punktwolken sein. Diese erlauben einen stark reduzierten Speicher- und Übertragungsaufwand im Vergleich zur Übertragung von kompletten Bildern. Weiterhin erlaubt die Verwendung dreidimensionaler Punktwolken eine besonders einfache Kombination von Daten mehrerer optischer Sensoren zu einer dreidimensionalen Darstellung. Vorteilhaft erlaubt die Verwendung dreidimensionaler Punktwolken weiterhin eine besonders einfache Bestimmung eines Abstands zwischen Objekten durch eine Bestimmung des Abstands der entsprechenden Punkte aus der Punktwolke.

Insbesondere kann die dreidimensionale Punktwolke durch eine automatisierte Vorfilterung auf relevante Teile des Überwachungsbereichs reduziert werden, beispielsweise auf Basis von erfassten Helligkeits-Kontrasten, sodass große einfarbige und planare Flächen, wie beispielsweise ein Fußboden, aus den ermittelten Sensordaten entfernt werden können.

Der detaillierte Aufbau einer erfindungsgemäßen Sensoreinheit mit einer Anzahl von optischen Sensoren zur Bereitstellung eine Abfolge von dreidimensionalen Darstellungen ist bekannt und derartige Sensoreinheiten sind bereits kommerziell erhältlich, sodass auf eine detaillierte Erläuterung der Funktionsweise dieser Sensoreinheit und des mindestens einen optischen Sensors aus der Anzahl von optischen Sensoren im Folgenden verzichtet wird.

Die früheren Auswertedaten umfassen Daten, die zu einem früheren Zeitpunkt als dem aktuellen Zeitpunkt erfasst wurden und für eine Auswertung der aktuellen Gefährdungssituation geeignet sind. Insbesondere können die früheren Auswertedaten auf früheren Positionen des zu schützenden Objektes und des gefährdenden Objektes eines jeweiligen Objektpaares relativ zueinander basieren.

Unter einem Alarm ist im Rahmen der Erfindung eine Ausgabe einer Information zu verstehen, die den Empfänger der Ausgabe über das Vorliegen eines Alarmzustandes informiert. Ein Alarm kann in diesem Sinne eine optische und/oder akustische Ausgabe und/oder eine Protokollierung des Alarmzustandes umfassen. Das erfindungsgemäße Auslösen eines Alarms kann in diesem Sinne auch das Ausgeben einer Alarminformation entsprechend des Alarmierungssignals umfassen, durch die ein Empfänger der Ausgabe über das Vorliegen eines Alarmzustandes informiert werden kann.

Der Überwachungsbereich für Objekte im medizinischen Umfeld ist beispielsweise ein für Patienten vorgesehener Bereich auf einer Krankenhausstation. In einem anderen Beispiel ist dieser Bereich ein für behandelte Personen vorgesehener Bereich innerhalb einer für betreutes Wohnen vorgesehenen Räumlichkeit.

Nachfolgend werden bevorzugte Ausführungsformen des erfindungsgemäßen Systems sowie weitere erfindungsgemäße Aspekte beschrieben.

In einer bevorzugten Ausführungsform ist die Zuordnung zwischen einem zu schützenden Objekt und einem gefährdenden Objekt abhängig von einer aktuellen Position des gefährdenden Objekts innerhalb des Überwachungsbereichs relativ zu dem zu schützenden Objekt. Hierdurch wird vermieden, dass eine unnötig große Anzahl von Objektpaaren durch das erfindungsgemäße System überwacht werden muss. So ist ein grundsätzlich als gefährdendes Objekt bekanntes Objekt, wie beispielsweise eine Hand, meist nur dann gefährlich, wenn sich dieses Objekt in der Nähe eines zu schützenden Objektes befindet oder sich auf das zu schützende Objekt zubewegt. Weiterhin ermöglicht die Zuordnung gemäß dieser Ausführungsform eine Bildung von Objektpaaren, bei der verschiedene zu schützende Objekte jeweils unterschiedlichen gefährdenden Objekten zu Objektpaaren zugeordnet sind. Hierdurch wird berücksichtigt, dass ein gefährdendes Objekt, welches sich in der Nähe eines ersten zu schützenden Objektes befindet für dieses eine ernsthafte Gefahr darstellen kann, während es für ein weit entferntes zweites zu schützendes Objekt möglicherweise derzeit keine Gefahr darstellt und kein entsprechendes Objektpaar überwacht werden braucht.

In einer besonders bevorzugten Ausführungsform sind die sich aus einer Auswertung früherer Auswertedaten, insbesondere früherer Positionen, ergebenden Parameter zumindest eine Distanz zwischen den Objekten eines Objektpaares und eine Richtung einer Bewegung der Objekte eines Objektpaares relativ zueinander. In dieser Ausführungsform kann besonders vorteilhaft auf eine zukünftige Distanz von zu schützendem Objekt und gefährdendem Objekt innerhalb eines Objektpaares zueinander geschlossen werden. So kann aus einer Veränderung der Distanz zwischen den Objekten auf eine Geschwindigkeit dieser Objekte zueinander geschlossen werden. In einer bevorzugten Variante ergibt sich für eine positive Geschwindigkeit mit der sich die Objekte aufeinander zu bewegen ein höherer aktueller Gefährdungswert als für eine niedrigere Geschwindigkeit. In einer weiteren Variante der Ausführungsform ist der aktuelle Gefährdungswert in der Art ausgebildet, dass wenn sich das zu schützende Objekt und das gefährdende Objekte voneinander wegbewegen, der Gefährdungsbetrag dieses Objektpaares im Vergleich zu einem früheren Gefährdungsbetrag stagniert oder abnimmt. Die Richtung der Bewegung wird vorzugsweise durch eine Auswertung der Veränderung der Distanz im Laufe der vergangenen erfassten Positionen bestimmt. In einer Variante dieser Ausführungsform wird als Distanz ein Abstand der geometrischen Schwerpunkte der beiden Objekt eines Objektpaares ermittelt. In einer alternativen Variante dieser Ausführungsform wird als Distanz ein Abstand der am dichtesten beieinanderliegenden Darstellungspunkte eines jeweiligen Objekts des Objektpaares ermittelt. In einer weiteren Variante dieser Ausführungsform wird aus der Distanz und der Bewegungsrichtung der beiden Objekte eines Objektpaares zueinander durch die Prozessoreinheit eine Berührungszeit berechnet, zu der es bei gleichbleibender Bewegung zu einer Berührung der beiden Objekte eines Objektpaares kommen würde. Die Berührungszeit bildet in dieser Variante zusätzlich einen sich aus der Auswertung früherer Auswertungsdaten ergebenden Parameter. Die Berechnung der Berührungszeit ist insbesondere vorteilhaft, wenn eines der Objekte zeitweise verdeckt für die Sensoreinheit ist, beispielsweise wenn das gefährdende Objekt sich hinter einem anderen Gegenstand, wie etwa unter einer Decke befindet. Angesichts einer aktuell berechneten Berührungszeit, die eine Vorhersage für eine Dauer bis zu einem zukünftigen Gefährdungsfall darstellt, kann bei einer derartigen zeitweisen Verdeckung des gefährdenden oder zu des zu schützenden Objekts dennoch rechtzeitig ein Alarmsignal ausgegeben und ein entsprechender Alarm ausgelöst werden.

In einer besonders vorteilhaften Ausführungsform wird jedem Objektpaar durch die Prozessoreinheit ein Wichtigkeitswert zugeordnet, der sich aus einem vorbestimmten Relevanzniveau des zu schützenden Objekts und/oder aus einem vorbestimmten Gefahrenniveau des gefährdenden Objekts ergibt, und wobei der aktuelle Gefährdungswert des Objektpaares abhängig von dem Wichtigkeitswert ist. Durch einen derartigen Wichtigkeitswert kann berücksichtigt werden, dass bestimmte Objektpaare zu einer sehr großen Gefahr für die Patientengesundheit führen können, wohingegen andere Objektpaare lediglich eine geringfügige Gefahr mit sich bringen. So kann beispielsweise einer aktuell zu verheilenden Wunde ein besonders hohes Relevanzniveau als zu schützendes Objekt zugeordnet werden, sodass jede Gefahr für diese Wunde und den entsprechenden Heilungsprozess schnell zu großen Gefährdungswerten und mithin einem hohen Gefährdungsbetrag und einer schnellen Alarmierung durch das Alarmierungssignal führt. Weiterhin kann eine Hand als gefährdendes Objekt ein höheres Gefahrenniveau zugeordnet werden als einem Kopf, sodass jedem Objektpaare mit der Hand grundsätzlich ein höherer Wichtigkeitswert zugeordnet wird, weil eine entsprechende Interaktion mit einem zu schützenden Objekt eine besonders große Gefahr für die Patientengesundheit darstellt. Die Zuordnung von Gefahrenniveau und Relevanzniveau erfolgt in einer bevorzugten Ausführungsform zumindest teilweise manuell. In einer alternativen oder ergänzenden Ausführungsform erfolgt die Zuordnung von Gefahrenniveau und Relevanzniveau durch eine vorbestimmte in einem Speicher der Prozessoreinheit hinterlegte Zuordnung zu vorbestimmten Objekten. Die Bestimmung des Wichtigkeitswerts aus Relevanzniveau und Gefahrenniveau ergibt sich beispielsweise aus einer Summe der beiden Niveaus, aus einem Produkt der beiden Niveaus, aus einem Mittelwert der beiden Niveaus, aus einem maximalen Wert der beiden Niveaus und/oder aus einer anderen geeigneten algebraischen Zuordnungsvorschrift. In einer alternativen Variante ergibt sich der Wichtigkeitswert direkt aus dem Relevanzniveau oder direkt aus dem Gefahrenniveau.

In einer zur vorherigen Ausführungsformen alternativen Ausführungsform ergibt sich der Wichtigkeitswert aus einer in einem Speicher der Prozessoreinheit hinterlegten Zuordnung von Wichtigkeitswert zu vorbestimmtem Objektpaar.

In einer weiteren vorteilhaften Ausführungsform ist der aktuelle Gefährdungswert eines Objektpaares auch abhängig davon, ob eine direkte Wegstrecke zwischen dem zu schützenden Objekt und dem gefährdenden Objekt durch einen weiteren Gegenstand versperrt ist. Hierdurch wird vorteilhaft berücksichtigt, dass ein Versperren der Wegstrecke zwischen den beiden Objekten eines Objektpaares dazu führt, dass die Gefahr einer Berührung der beiden Objekte zumindest für einen gewissen Zeitraum sehr gering ist. Entsprechend führt das Versperren der Wegstrecke vorzugsweise zu einem aktuellen Gefährdungswert, der zu einer Reduzierung des bestimmten Gefährdungsbetrags führt.

In einer bevorzugten Ausführungsform wird die Überwachung eines Objektpaares durch die Prozessoreinheit beendet, falls der verrechnete Gefährdungsbetrag unter einen unteren Schwellwert fällt. Der untere Schwellwert ist hierbei derart ausgebildet, dass keine Gefahr für die Patientengesundheit durch das ursprünglich gefährdende Objekt besteht.

Besonders vorteilhaft ist die Festlegung der Anzahl zu schützender Objekte und/oder der gefährdenden Objekte durch eine automatisierte Objekterkennung realisiert. Verfahren zur automatisierten Objekterkennung sind allgemein bekannt, sodass sie im Folgenden nicht detailliert erläutert werden. Besonders zuverlässig können durch bekannte automatisierte Algorithmen zur Objekterkennung vorbestimmte Objekte erkannt werden. Hierdurch ist es besonders vorteilhaft, gängige zu schützende medizinische Objekte aus dem medizinischen Umfeld vor der Nutzung des erfindungsgemäßen Systems zu definieren, wie beispielsweise einen Tubus, einen Katheter, ein Versorgungskabel, Verbände und/oder medizinische Geräte. Erkennt die Prozessoreinheit ein derartiges bereits definiertes Objekt in dem Überwachungsbereich, kann es automatisch als zu schützendes Objekt festgelegt werden.

In einer weiteren vorteilhaften Ausführungsform ist die Festlegung der Anzahl zu schützender Objekte zumindest teilweise durch eine Interaktion mit einem Nutzer des Systems realisiert. In einer bevorzugten Variante handelt es sich bei der Interaktion mit dem Nutzer um eine manuelle Definition des zu schützenden Objektes, beispielsweise durch ein Markieren dieses Objektes auf einer grafischen Benutzeroberfläche, insbesondere durch ein Markieren durch Mausklick oder durch Berührung. In einer weiteren vorteilhaften Variante handelt es sich bei der Interaktion mit dem Nutzer um eine Gestensteuerung, beispielsweise indem innerhalb des Überwachungsbereichs medizinisches Personal durch eine vorbestimmte Geste die Aktivierung der Überwachung für ein durch die Geste definiertes Objekt auslöst. In einer weiteren vorteilhaften Variante handelt es sich bei der Interaktion um eine vorbestimmte Behandlungsaktion innerhalb des Überwachungsbereich, sodass beispielsweise durch die Prozessoreinheit ein Verband als zu schützendes Objekt dadurch erkannt wird, dass dieser angelegt wird, oder dass ein Tubus als zu schützendes Objekt dadurch erkannt wird, dass dieser bei der Integrierung in einen Patienten verwendet wird. Sämtliche Varianten dieser Ausführungsform haben den Vorteil, dass selbst für ungewöhnliche Formen und Arten von zu schützenden Objekten, die möglicherweise nicht durch automatische Objekterkennung erfasst werden können, eine Festlegung als zu schützendes Objekt durch den Nutzer möglich ist. Weiterhin kann in dieser Ausführungsform durch den Nutzer sichergestellt werden, dass kein zu schützendes Objekt durch die Prozessoreinheit übersehen wird, was besonders vorteilhaft für die Patientensicherheit ist.

In einer Ausführungsform des erfindungsgemäßen Systems ist das zeitliche Auswertungsintervall dasjenige Zeitintervall, das durch einen Bestimmungszeitpunkt für die Bestimmung des entsprechenden Objektpaares und dem aktuellen Zeitpunkt gebildet ist. In dieser Ausführungsform werden vorteilhaft sämtliche Gefährdungswerte berücksichtigt, die seit dem Zuordnen der Objekte zu einem Objektpaar ermittelt wurden. Hierdurch kann durch die besonders genaue qualitative Auswertung des Überwachungsbereichs über die Zeit besonders vertrauenswürdig auf ein zukünftiges Verhalten der Objekte eines Objektpaares relativ zueinander geschlossen werden.

In einer vorteilhaften Ausführungsform weist das erfindungsgemäße System weiterhin eine Benutzerschnittstelle auf, die mit der Prozessoreinheit verbunden ist, und die ausgebildet ist, eine manuelle Steuerung der Festlegung eines zu schützenden Objekts, der Erkennung eines gefährdenden Objekts und/oder der Zuordnung der beiden Objekte zu einem Objektpaar zu ermöglichen. Eine derartige Benutzerschnittstelle kann beispielsweise durch eine Tastatur, ein Touchdisplay, eine Computermaus, einen optischen Sensor und/oder einen Joystick realisiert sein. Die Bedienung der Prozessoreinheit durch die Benutzerschnittstelle gemäß dieser Ausführungsform ermöglicht vorteilhaft eine besonders zuverlässige Festlegung des zu schützenden Objekts und/oder eine besonders zuverlässige Erkennung des gefährdenden Objekts und/oder eine besonders zuverlässiger Zuordnung der beiden Objekte zu einem Objektpaar. Insbesondere werden Fehler vermieden, die bei der automatisierten Festlegung, Erkennung und/oder Zuordnung erfolgen können.

Erfindungsgemäß ist das zu schützende Objekt mindestens ein Objekt aus der Gruppe: Infusion, Medizingerät, Kabel, Schlauch, Tubus, Verband, Wunde. Derartige Objekte bilden im Klinikalltag regelmäßig zu schützende Objekte, sodass deren manuelle und/oder automatische Festlegung als zu schützendes Objekt besonders vorteilhaft ist. Als Person ist in diesem Zusammenhang beispielsweise der gesamte Patient zu verstehen, sodass in diesem Fall eine Alarmierung bei jeglichem Kontakt mit dem Patienten, insbesondere durch Unbefugte, vorgesehen ist.

In einer besonders vorteilhaften Ausführungsform des erfindungsgemäßen Systems, ist die Sensoreinheit weiterhin ausgebildet, Sensordaten, die ein festgelegtes aktuell zu schützendes Objekt betreffen, seltener im Rahmen des Sensorsignals an die Prozessoreinheit auszugeben, als Sensordaten, die ein erkanntes aktuell gefährdendes Objekt betreffen. In dieser Ausführungsform wird vorteilhaft ausgenutzt, dass es sich bei den zu schützenden Objekten, wie beispielsweise bei der Gruppe von Objekten gemäß der vorhergehenden Ausführungsform, um Objekte handelt, die typischerweise im klinischen Umfeld keine oder nur eine sehr langsame Bewegung ausführen. Hierdurch kann in dieser Ausführungsform vorteilhaft ein Datenstrom zwischen Sensoreinheit und Prozessoreinheit und/oder ein Speicherplatz für empfangene Sensordaten verringert werden.

In einer weiteren vorteilhaften Ausführungsform ist die Prozessoreinheit ausgebildet, das empfangene Sensorsignal zu filtern und nur das gefilterte Signal zur weiteren Verarbeitung, insbesondere zur weiteren Überwachung von Objektpaaren, zu verwenden. Hierbei werden in dem gefilterten Signal vorzugsweise nur Bereiche des Überwachungsbereichs indiziert, in denen seit dem letzten Zeitschritt eine Bewegung oder eine Veränderung des Überwachungsbereichs stattgefunden hat.

Erfindungsgemäß ist das gefährdende Objekt mindestens ein Objekt aus der Gruppe: Hand eines Patienten, Fuß eines Patienten, Kopf eines Patienten, Tier, Bettgitter. Derartige Objekte lassen sich vorteilhaft besonders zuverlässig automatisiert erkennen. Weiterhin können die genannten Objekte im klinischen Umfeld zu einer Interaktion mit einem zu schützenden Objekt führen, die regelmäßig eine Gefahr für die Patientengesundheit darstellt.

In einer besonders vorteilhaften Ausführungsform indiziert das Alarmierungssignal das zu schützende Objekt und/oder das gefährdende Objekt. In einer Variante dieser Ausführungsform indiziert der ausgelöste Alarm das zu schützende Objekte und/oder das gefährdende Objekt. Hierdurch kann ein Nutzer des erfindungsgemäßen Systems bereits beim Wahrnehmen eines durch das Alarmierungssignals ausgelösten Alarms die vorliegende Gefahrensituation erfassen und eventuell besonders schnell Vorkehrungen zur Sicherung der Patientengesundheit treffen.

Besonders vorteilhaft können verschiedene das Alarmierungssignal auslösende Objektpaare auch zu verschiedenen Arten der Alarmierung führen. So kann beispielsweise ein durch eine fremde Person als gefährdendes Objekt ausgelöster Alarm zumindest zusätzlich bei einem Sicherheitsdienst des entsprechenden Krankenhauses angezeigt werden. In einem anderen Beispiel wird ein durch ein Bettgitter als gefährdendes Objekt ausgelöster Alarm nur innerhalb des entsprechenden Zimmers als Alarm ausgegeben. In einem anderen Beispiel wird ein durch ein Tier als gefährdendes Objekt ausgelöster Alarm vorteilhaft innerhalb der gesamten entsprechenden Krankenstation ausgegeben, da dieses Tier auch für andere Patienten eine Gefahr darstellen könnte.

In einer weiteren Ausführungsform ist die Prozessoreinheit ausgebildet, ein weiteres entsprechendes Alarmierungssignal auszugeben, falls der so verrechnete Alarmierungsbetrag des Objektpaares einen weiteren vorbestimmten Schwellenwert übersteigt, wobei der weitere vorbestimmte Schwellenwert größer als der vorbestimmte Schwellenwert ist. Hierdurch können entsprechend der Gefahr, die aktuell für die Patientengesundheit vorliegt, verschiedene Alarmstufen ausgelöst werden. So wird beispielsweise ein lokaler nur im Patientenzimmer wahrnehmbarer Alarm bei Erreichen des vorbestimmten Schwellenwertes und ein globaler auch in einem Aufenthaltsbereich für medizinisches Personal wahrnehmbarer Alarm bei Erreichen des weiteren vorbestimmten Schwellenwertes ausgegeben.

**In** einer weiteren erfindungsgemäßen Ausführungsform löst das Alarmierungssignal eine Protokollierung der aktuell vorliegenden Gefahrensituation innerhalb eines entsprechenden Speichers des erfindungsgemäßen Systems aus. Hierdurch können vorteilhaft Beeinträchtigungen der Patientengesundheit aufgrund einer früheren Gefahrensituation rückblickend nachvollzogen werden.

In einer bevorzugten Ausführungsform des erfindungsgemäßen Systems löst das Alarmierungssignal entsprechend des das Alarmierungssignal auslösenden Objektpaares einen Alarm bei dem zu schützenden Objekt und/oder bei dem gefährdenden Objekt aus. In einer Variante dieser Ausführungsform führt der Alarm bei dem zu schützenden Objekt zu einer Anpassung dieses Objektes, die die Gefahr für die Patientengesundheit reduziert. Beispielsweise wird in einer Variante ein medizinisches Gerät als zu schützendes Objekt dahingehend angepasst, dass es keine versehentliche Eingabe durch den Nutzer entgegennimmt. In einem anderen Beispiel wird als Alarm eine Audio-Nachricht an die das gefährdende Objekt bildende Person versendet, sodass diese über das Risiko für die Patientengesundheit informiert wird.

Gemäß einem weiteren Aspekt der Erfindung wird die oben genannte Aufgabe gelöst durch ein Verfahren zur automatischen Erkennung von Gefährdungssituationen innerhalb eines Überwachungsbereichs für Objekte im medizinischen Umfeld.

Das erfindungsgemäße Verfahren weist dabei die folgenden Schritte auf:

- Bestimmen eines Sensorsignals, das Sensordaten umfasst, die eine Abfolge von dreidimensionalen Darstellungen des Überwachungsbereichs über eine Überwachungsdauer indizieren, und Ausgeben des Sensorsignals in Echtzeit,
- Empfangen des Sensorsignals,

- Festlegen einer Anzahl zu schützender Objekte innerhalb des Überwachungsbereichs anhand der indizierten dreidimensionalen Darstellungen,
- Erkennen, insbesondere automatisiertes Erkennen von gefährdenden Objekten innerhalb des Überwachungsbereichs anhand der indizierten dreidimensionalen Darstellungen,
- Bestimmen einer Anzahl von Objektpaaren durch eine Zuordnung zwischen einem zu schützenden Objekt aus der Anzahl zu schützender Objekte und einem gefährdenden Objekt,
- Zuordnen eines aktuellen Gefährdungswertes zu einem jeweiligen Objektpaar abhängig von den aktuellen Positionen des zu schützenden Objekts und des gefährdenden Objekts und von einer Gruppe von sich aus einer Auswertung früherer Auswertedaten ergebender Parameter, wobei die sich ergebenden Parameter zumindest eine Distanz zwischen den Objekten eines Objektpaares und eine Richtung einer Bewegung der Objekte eines Objektpaares relativ zueinander sind,- Verrechnen der aktuellen Gefährdungswerte für ein jeweiliges Objektpaar zu einem jeweiligen Gefährdungsbetrag über ein zeitliches Auswertungsintervall bis zum aktuellen Zeitpunkt,
- Ausgeben eines entsprechenden Alarmierungssignals, falls der so bestimmte Gefährdungsbetrag einen vorbestimmten Schwellenwert übersteigt,
- Empfangen des Alarmierungssignals und Auslösen eines Alarms abhängig von dem Alarmierungssignal.

Vorteilhaft erlaubt das erfindungsgemäße Verfahren ein besonders schnelles Erkennen einer Gefährdungssituation unter Vermeidung von Fehlalarmen.

Insbesondere das Verrechnen der aktuellen Gefährdungswerte für ein jeweiliges Objektpaar über das zeitliche Auswertungsintervall ist vorteilhaft, da durch ein Berücksichtigen der jüngeren Vergangenheit des Überwachungsbereiches besonders zuverlässig auf eine zukünftige Entwicklung der Positionen eines jeweiligen Objektpaare geschlossen werden kann.

Weiterhin ist vorteilhaft, dass die gefährdenden Objekte vorzugsweise automatisiert erkannt werden, da hierdurch sichergestellt werden kann, dass nicht gefährdende Objekte während einer beispielsweise manuellen Eingabe dieser Objekte übersehen werden.

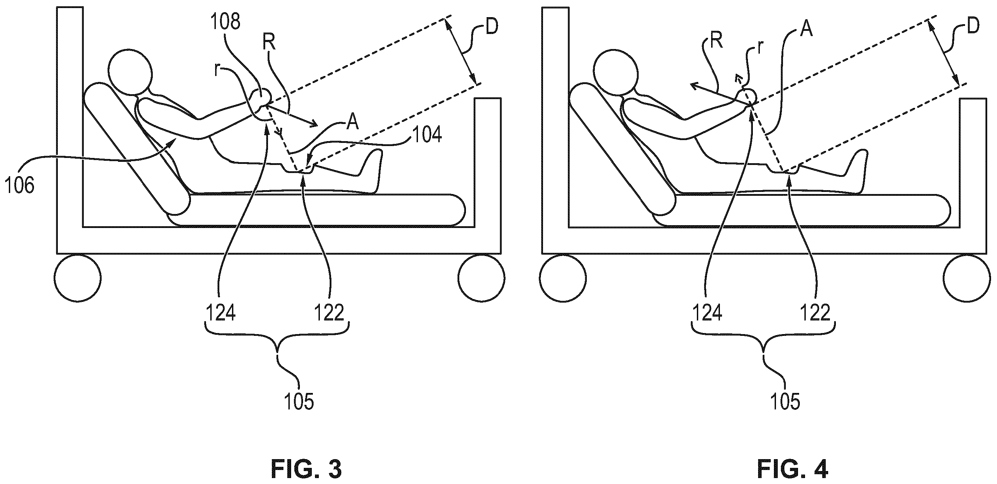

In einer besonders bevorzugten Ausführungsform des erfindungsgemäßen Verfahrens umfasst das Verrechnen der aktuellen Gefährdungswerte ein Aufsummieren dieser aktuellen Gefährdungswerte bis zum aktuellen Zeitpunkt zu dem jeweiligen Gefährdungsbetrag des Objektpaares. Das Aufsummieren stellt eine besonders einfache mathematische Operation dar, die eine besonders schnelle Verarbeitung durch die Prozessoreinheit durch ein besonders schnelles Verrechnen der aktuellen Gefährdungswerte ermöglicht. In einer bevorzugten Variante dieser Ausführungsform kann der aktuelle Gefährdungswert auch negativ sein, sodass der aufsummierte Gefährdungsbetrag abhängig von den Aktionen innerhalb des Überwachungsbereich auch wieder abnehmen kann, beispielsweise wenn sich gefährdendes Objekt und zu schützendes Objekt nach einer anfänglichen Annäherung wieder voneinander entfernen. Eine detaillierte Beschreibung, wie eine derartige Bestimmung des Gefährdungsbetrages realisiert sein kann, befindet sich in der Beschreibung zu Figs. 3 bis 5.

In einer weiteren Ausführungsform des erfindungsgemäßen Verfahrens, wird dieses Verfahren gestoppt, falls medizinisches Personal durch die Prozessoreinheit innerhalb des Überwachungsbereichs detektiert wird. Eine solche Detektion kann beispielsweise durch das Auslesen einer durch das medizinische Personal mitgeführten Identifikationsnummer erfolgen. Vorzugsweise wird das Verfahren gemäß dieser Ausführungsform zusammen mit den bisher ermittelten Gefährdungswerten weitergeführt, sobald das medizinische Personal den Überwachungsbereich wieder verlassen hat.

Weiterhin wird die oben genannte Aufgabe gelöst durch ein Programm mit einem Programmcode zur Durchführung des Verfahrens gemäß mindestens einer der oben angegebenen Ausführungsformen des erfindungsgemäßen Verfahrens, wenn der Programmcode auf einem Computer, einem Prozessor oder einer programmierbaren Hardwarekomponente ausgeführt wird.

Das Programm kann dabei auch nur einen Teil der erfindungsgemäßen Datenverarbeitung ausführen. Vorzugsweise werden zumindest die Funktion der Prozessoreinheit und der Ausgabeeinheit durch ein Programm und/oder aufeinander abgestimmte Teile des Programms gesteuert. Insbesondere die Verarbeitung der Sensordaten zu dem Sensorsignal wird in erfindungsgemäßen Ausführungsformen des Programms durch einen separaten Teil des Programms innerhalb eines Prozessors der Sensoreinheit gesteuert. Vorzugsweise wird das erfindungsgemäße Programm von einem Prozessor des erfindungsgemäßen Systems ausgeführt. Alternativ wird das Programm zumindest durch einen ersten Prozessor der Prozessoreinheit und durch einen zweiten Prozessor der Ausgabeeinheit ausgeführt.

Die Erfindung soll nun anhand von in den Figuren schematisch dargestellten, vorteilhaften Ausführungsbeispielen näher erläutert werden. Von diesen zeigen im Einzelnen:

Fig. 1          eine schematische Darstellung eines ersten Ausführungsbeispiels eines erfindungsgemäßen Systems;
Fig. 2          eine schematische Darstellung eines zweiten Ausführungsbeispiels des erfindungsgemäßen Systems;
Figs. 3, 4, 5   schematische Illustrationen verschiedener Gefährdungssituationen, wobei sich zu schützendes Objekt und gefährdendes Objekt einander annähern (Fig. 3), wobei sich zu schützendes Objekt und gefährdendes Objekt voneinander wegbewegen (Fig. 4), und wobei sich zwischen zu schützendem Objekt und gefährdendem Objekt ein weiterer Gegenstand befindet (Fig. 5);

Fig. 6        ein Flussdiagramm eines ersten Ausführungsbeispiels eines erfindungsgemäßen Verfahrens.

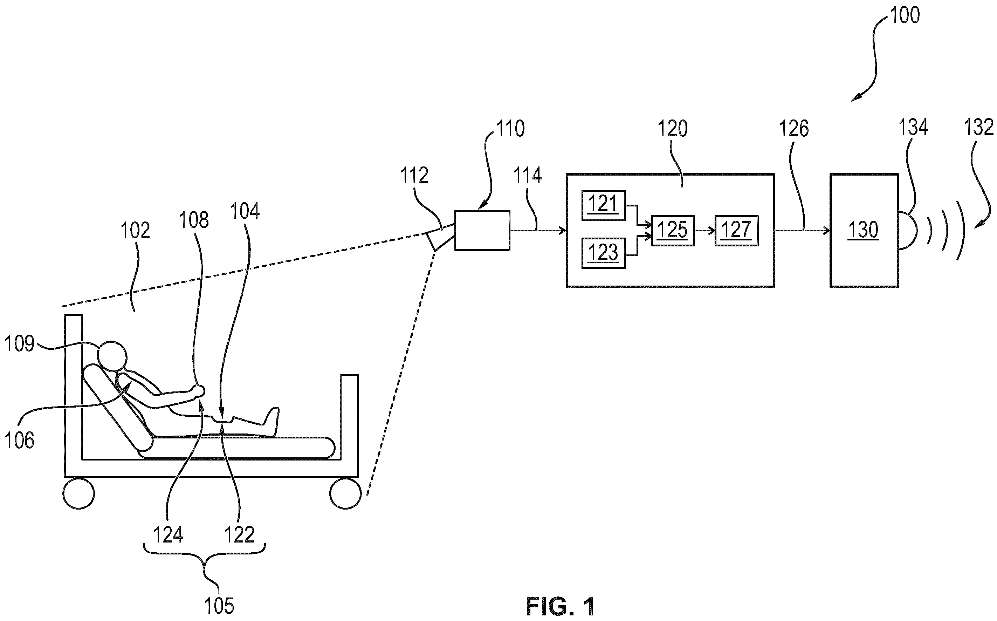

Fig. 1 zeigt eine schematische Darstellung eines ersten Ausführungsbeispiels eines erfindungsgemäßen Systems 100.

Das System 100 zur automatischen Erkennung von Gefährdungssituationen innerhalb eines Überwachungsbereichs 102 für Objekte im medizinischen Umfeld, umfasst eine Sensoreinheit 110, eine Prozessoreinheit 120 und eine Ausgabeeinheit 130.

Die Sensoreinheit 110 weist eine Anzahl von optischen Sensoren 112 auf und ist ausgebildet, ein Sensorsignal 114 zu bestimmen und in Echtzeit auszugeben. Dabei ist die Anzahl von optischen Sensoren 112 weiterhin derart in dem medizinischen Umfeld anordnenbar und derart ausgebildet, dass das Sensorsignal 114 Sensordaten umfasst, die eine Abfolge von dreidimensionalen Darstellungen des Überwachungsbereichs 102 während einer Überwachungsdauer indizieren. In dem dargestellten Ausführungsbeispiel ist die Anzahl von optischen Sensoren 112 innerhalb eines Gehäuses verbaut, wobei durch die unterschiedliche Lage der optischen Sensoren 112 eine dreidimensionale Darstellung des Überwachungsbereichs 102 durch die Sensordaten indiziert wird. In einem nicht dargestellten Ausführungsbeispiel ist der mindestens eine Sensor aus der Anzahl von optischen Sensoren dazu ausgebildet, über eine time-of-flight Messung innerhalb des Überwachungsbereichs die dreidimensionalen Darstellungen zu bestimmen. In einem weiteren nicht dargestellten Ausführungsbeispiel ist der mindestens eine Sensor aus der Anzahl von optischen Sensoren dazu ausgebildet, über eine sogenannten structured-light Messung, also eine Ausgabe eines bekannten Strahlungsmusters im nicht sichtbaren Wellenlängenbereich und einen Empfang von entsprechenden Reflexionen, die dreidimensionalen Darstellungen zu bestimmen.

Die Ausgabe des Sensorsignals 114 an die Prozessoreinheit 120 erfolgt in dem dargestellten Ausführungsbeispiel drahtlos über eine Funkverbindung, beispielsweise über WLAN, Bluetooth, BLE oder ZigBee. In einem nicht dargestellten Ausführungsbeispiel erfolgt die Ausgabe des Sensorsignals Kabel-basiert, beispielsweise im Rahmen eines Bus-Systems, insbesondere eines Ethernet-Systems.

Die Prozessoreinheit 120 ist ausgebildet, das Sensorsignal 114 zu empfangen und in einem ersten Verarbeitungsschritt 121 anhand der indizierten dreidimensionalen Darstellungen eine Festlegung einer Anzahl zu schützender Objekte 122 innerhalb des Überwachungsbereichs 102 auszuführen. Vorliegend wird lediglich eine Wunde 104 an dem Bein des Patienten 106 als zu schützendes Objekt 122 festgelegt. Diese Festlegung erfolgte vorliegend durch eine Gestengesteuerte Erkennung der Wunde 104 durch die Prozessoreinheit 120. Hierbei wurde durch die Prozessoreinheit 120 automatisiert erkannt, dass die Wunde 104 behandelt und durch einen Verband (nicht dargestellt) geschützt wurde, sodass es sich bei dem Bereich der Wunde 104 um ein zu schützendes Objekt handelt. In einem weiteren nicht dargestellten Ausführungsbeispiel erfolgt eine automatisierte Erkennung des zu schützenden Objekts durch einer Auswertung der Sensordaten derart, dass ein Objekt aus der nachfolgend genannten Gruppe von vorbestimmten Objekten automatisch als zu schützendes Objekt festgelegt wird oder als zu schützendes Objekt einem Nutzer des Systems automatisch vorgeschlagen wird. Diese Gruppe von vorbestimmten Objekten umfasst mindestens: Infusion, Medizingerät, Kabel, Schlauch, Tubus, Verband, Wunde.

Weiterhin ist die Prozessoreinheit 120 ausgebildet, in einem zweiten Verarbeitungsschritt 123 anhand der indizierten dreidimensionalen Darstellungen eine automatisierte Erkennung von gefährdenden Objekten 124 innerhalb des Überwachungsbereichs 102 auszuführen. Vorliegend werden die Hand 108 und der Kopf 109 des Patienten 106 als gefährdende Objekte erkannt. Für die automatisierte Erkennung wurde der Überwachungsbereich durch die Prozessoreinheit 120 durchsucht nach Objekten, die zu einer vorbestimmten und in einem Speicher der Prozessoreinheit 120 hinterlegten Gruppe von gefährdenden Objekten gehören. Diese Gruppe umfasst mindestens die Objekte: Fuß eines Patienten 106, Bettgitter, Hand 108 eines Patienten 106, Kopf eines Patienten 106, Tier. In einem nicht dargestellten erfindungsgemäßen Ausführungsbeispiel werden die gefährdenden Objekte durch eine manuelle Auswahl auf einer Benutzeroberfläche einer Benutzerschnittstelle des Systems festgelegt und dadurch von der Prozessoreinheit erkannt.

Nach dem Zwischenspeichern von zu schützenden Objekten 122 und gefährdenden Objekten 124 bestimmt die Prozessoreinheit 120 in einem Zuordnungsschritt 125 eine Anzahl von Objektpaaren 105 durch eine Zuordnung zwischen einem zu schützenden Objekt 122 aus der Anzahl zu schützender Objekte und einem gefährdenden Objekt 124. Dabei erkennt die Prozessoreinheit 120, dass der Kopf 109 des Patienten 106 nicht in die Nähe der Wunde 104 gebracht werden kann und mithin keine Gefahr für die Wunde 104 als zu schützendes Objekt darstellt. Daher bestimmt die Prozessoreinheit 120 die Kombination aus Hand 108 und Wunde 104 als einziges relevantes und daher zu überwachendes Objektpaar 105.

Weiterhin ist die Prozessoreinheit 120 ausgebildet, im Laufe der Überwachungsdauer im Rahmen eines Überwachungsvorgangs 127 die Positionen von zu schützendem Objekt 122 und gefährdendem Objekt 124 des in diesem Ausführungsbeispiel einzigen überwachten Objektpaares 105 innerhalb des Überwachungsbereichs 102 zu überwachen und abhängig von den beiden aktuellen Positionen und einer Gruppe von sich aus einer Auswertung früherer Auswertedaten ergebender Parameter einem jeweiligen Objektpaar 105 einen aktuellen Gefährdungswert zuzuordnen. Schließlich werden über ein zeitliches Auswertungsintervall für jedes Objektpaar 105 jeweils die aktuellen Gefährdungswerte bis zum aktuellen Zeitpunkt verrechnet und ein entsprechendes Alarmierungssignal 126 ausgegeben, falls ein so verrechneter Gefährdungsbetrag eines Objektpaares 105 einen vorbestimmten Schwellenwert übersteigt. Ein konkretes Ausführungsbeispiel für ein derartiges Verrechnen der Gefährdungswerte ist im Rahmen der Figuren 3 bis 5 beschrieben.

In dem dargestellten Ausführungsbeispiel erfolgt die Ausgabe des Alarmierungssignals 126 Kabel-basiert. In einem nicht dargestellten Ausführungsbeispiel erfolgt diese Ausgabe durch eine kabellose Verbindung, wie sie im Bereich der Kommunikationstechnik in verschiedenen Varianten bekannt ist.

Die Ausgabeeinheit 130 ist ausgebildet, das Alarmierungssignal 126 zu empfangen und abhängig von dem Alarmierungssignal 126 einen Alarm 132 auszulösen. Bei dem Alarm handelt es sich in dem dargestellten Ausführungsbeispiel um einen akustischen Alarm, der durch einen Lautsprecher 134 bereitgestellt wird. In einem nicht dargestellten alternativen oder ergänzenden Ausführungsbeispiel wird ein optischer Alarm durch die Ausgabeeinheit ausgelöst. In einem weiteren nicht dargestellten Ausführungsbeispiel wird eine Protokollierung der Gefahrensituation durch die Ausgabeeinheit ausgelöst. In einem weiteren nicht dargestellten Ausführungsbeispiel wird der Alarm durch ein externes Gerät, das nicht zum erfindungsgemäßen System gehört, ausgegeben, wobei jedoch die Ausgabeeinheit 130 dazu ausgebildet ist, den entsprechenden Alarm durch ein Auslösesignal an dem externen Gerät auszulösen.

In dem dargestellten Ausführungsbeispiel weisen sämtliche Einheiten des erfindungsgemäßen Systems 100 separate Gehäuse auf, da sämtliche Einheiten beabstandet voneinander angeordnet sind. Hierdurch ist es beispielsweise möglich, die Prozessoreinheit an einem zentralen Ort im medizinischen Umfeld vorzusehen und eine Anzahl von erfindungsgemäßen Systemen mit einer entsprechenden Anzahl von verschiedenen Sensoreinheiten mit einer gemeinsamen Prozessoreinheit zu betreiben.

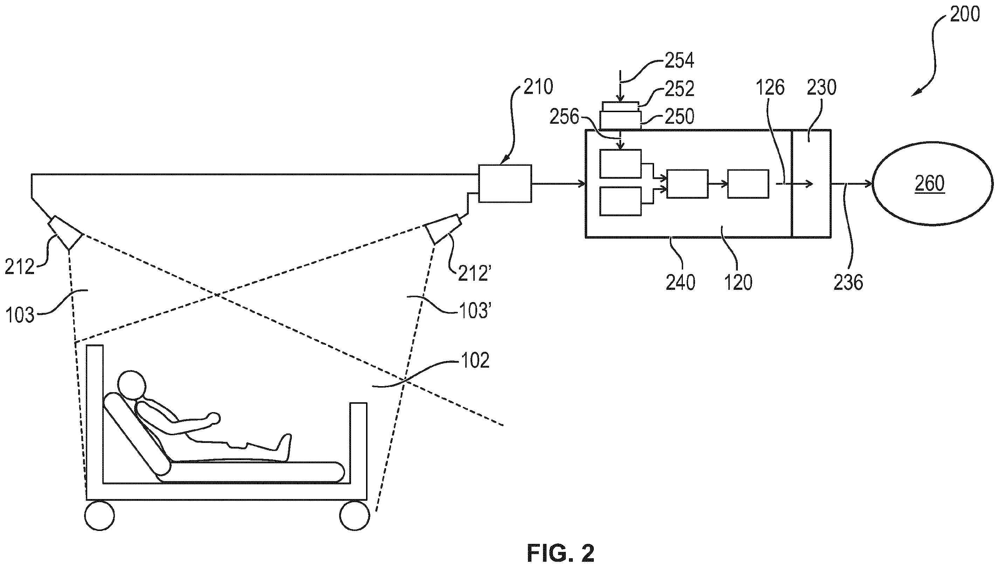

Fig. 2 zeigt eine schematische Darstellung eines zweiten Ausführungsbeispiels des erfindungsgemäßen Systems 200.

Das System 200 unterscheidet sich von dem in Fig. 1 dargestellten System 100 unter anderem dadurch, dass die Sensoreinheit 210 zwei separate Kameras mit einer entsprechenden Anzahl von optischen Sensoren 212, 212' und zwei Teilüberwachungsbereichen 103, 103' umfasst. Eine kombinierte Verarbeitung der entsprechend erfassten Sensordaten ist dadurch möglich, dass die Teilüberwachungsbereiche 103, 103' einen gemeinsamen zu überwachenden Überwachungsbereich 102 als Schnittmenge aufweisen. Hierdurch wird eine einzige Abfolge von dreidimensionalen Darstellungen dieses gemeinsamen zu überwachenden Überwachungsbereichs 102 indiziert.

Weiterhin unterscheidet sich das System 200 dadurch von dem System 100 aus Fig. 1, dass Prozessoreinheit 120 und Ausgabeeinheit 230 in einem gemeinsamen Gehäuse 240 angeordnet sind. Das Gehäuse 240 umfasst zudem eine Benutzerschnittstelle 250 als zusätzlichen Bestandteil des Systems 200.

Die Benutzerschnittstelle 250 ist mit der Prozessoreinheit 120 signaltechnisch verbunden. Die Benutzerschnittstelle 250 ist dabei dazu ausgebildet, eine manuelle Steuerung der Festlegung eines zu schützenden Objekts, der Erkennung eines gefährdenden Objekts und/oder der Zuordnung der beiden Objekte zu einem Objektpaar zu ermöglichen. Hierfür weist die Benutzerschnittstelle 250 ein Touchdisplay 252 zum Empfang einer Benutzereingabe 254 auf. Vorliegend erfolgt die Festlegung eines zu schützenden Objektes derart, dass der aktuelle Überwachungsbereich 102 auf dem Touchdisplay 252 dargestellt wird, und das zu schützende Objekt durch ein Berühren dieses Objektes auf dem Touchdisplay 252 festgelegt werden kann. Analog erfolgt die manuelle Erkennung und Speicherung eines gefährdenden Objektes. Ein manuelles Zuordnen zweier Objekte zu einem Objektpaar über das Touchdisplay 252 kann beispielsweise durch eine Wisch-Geste eines Objektes über ein anderes, durch ein gleichzeitiges Berühren beider Objekte, durch ein schnell aufeinanderfolgendes Antippen zweier Objekte auf dem Touchdisplay, und/oder durch eine entsprechende Notiz in einer Datenbank der Benutzerschnittstelle 250 erfolgen. In einem nicht dargestellten Ausführungsbeispiel ist die Benutzerschnittstelle als Tastatur, als Computermaus, als optischer Sensor und/oder als Joy Stick ausgebildet. Vorliegend wird durch die Benutzerschnittstelle 250 entsprechend der Benutzereingabe 254 ein entsprechendes Eingabesignal 256 an die Prozessoreinheit 120 ausgegeben.

In dem dargestellten Ausführungsbeispiel ist die Ausgabeeinheit 230 Kabel-basiert mit der Prozessoreinheit 120 verbunden. Anders als bei dem System 100 aus Fig. 1, ist die Ausgabeeinheit 230 dazu ausgebildet, entsprechend des empfangenen Alarmierungssignals 126 ein Auslösesignal 236 an ein Kommunikationsnetzwerk 260 des Krankenhauses auszugeben und innerhalb dieses Kommunikationsnetzwerks 260 einen Alarm auszulösen. Sowohl das Alarmierungssignal 126, als auch das entsprechend ausgegebene Auslösesignal 236 indizieren das zu schützende Objekt und/oder das gefährdende Objekt. Hierdurch kann durch das Kommunikationsnetzwerk 260 ein Alarm ausgelöst werden, der speziell an die entstandene Gefährdungssituation angepasst ist. So kann beispielsweise lediglich ein Alarmgeber innerhalb des betreffenden Krankenhauszimmers ausgelöst werden, oder es kann in einem schwerwiegenden Fall, wie beispielsweise bei einer Gefährdung durch ein Tier eine Gruppe von Alarmgebern verteilt über eine Krankenhausstation ausgelöst werden.

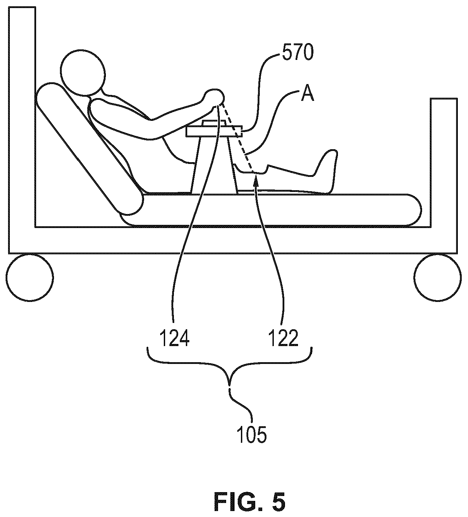

Figuren 3, 4 und 5 zeigen schematische Illustrationen verschiedener Gefährdungssituationen, wobei sich zu schützendes Objekt 122 und gefährdendes Objekt 124 einander annähern (Fig. 3), wobei sich zu schützendes Objekt 122 und gefährdendes Objekt 124 voneinander wegbewegen (Fig. 4), und wobei sich zwischen zu schützendem Objekt 122 und gefährdendem Objekt 124 ein weiterer Gegenstand 570 befindet (Fig. 5).

Anhand der in den Figuren 3 bis 5 dargestellten Gefährdungssituationen sollen im Folgenden die Zuordnung des jeweils gleichen Objektpaares 105 zu einem aktuellen Gefährdungswerte und die Verrechnung der aktuellen Gefährdungswerte zu einem Gefährdungsbetrag gemäß dem in Fig. 1 dargestellten bevorzugten Ausführungsbeispiel erläutert werden.

In den drei dargestellten Gefährdungssituationen handelt es sich bei dem zu schützenden Objekt 122, wie bereits im Rahmen der Ausführungsbeispiele aus Fig. 1 und 2 erläutert, um eine Wunde 104 am Knie des Patienten 106. Das gefährdende Objekt 124 ist wiederum die Hand 108 des Patienten 106 und das betrachtete Objektpaar ist mithin durch die Wunde 104 und die Hand 108 des Patienten 106 gebildet.

Der diesem Objektpaar 105 zum aktuellen Zeitpunkt zugeordnete aktuelle Gefährdungswert $g(t, \vec{x}, p_1, ..., p_n)$ ist abhängig von dem aktuellen Zeitschritt t, dem aktuellen Abstandsvektor $\vec{x}$, der den Betrag des Abstandes D beider Objekte des Objektpaares aufweist und entlang einer durch die aktuelle Position des zu schützenden Objektes 122 und die aktuelle Position des gefährdenden Objektes 124 definierten Richtung ausgerichtet ist. Weiterhin ist der Gefährdungswerte g abhängig von mehreren Parametern $p_1, ..., p_n$, die sich aus der Auswertung früherer Auswertungsdaten ergeben. Bei diesen Parametern handelt es sich vorzugsweise um den Betrag D des aktuellen Abstandsvektors und/oder eine aktuelle Bewegungsrichtung R der beiden Objekte relativ zueinander und/oder um einen durch die Prozessoreinheit dem Objektpaare 105 zugeordneten Wichtigkeitswert und/oder um einen Parameter der anzeigt, ob zwischen dem gefährdenden Objekt 124 und dem zu schützenden Objekt 122 eine freie Wegstrecke vorliegt.

Als aktuelle Position des jeweiligen Objekts wird der geometrische Schwerpunkt dieses Objekts durch die Prozessoreinheit 120 verwendet. In einem nicht dargestellten Ausführungsbeispiel wird der Abstandsvektor durch das am dichtesten beieinanderliegende Punktepaar von Sensordaten-Punkten des jeweiligen Objektes gebildet. Vorzugsweise wird der geometrische Schwerpunkt des Objektes nicht mit jedem neuen Datensatz von Sensordaten neu berechnet, besonders bevorzugt wird dieser Schwerpunkt nur einmal für ein Objekt innerhalb des Überwachungsbereichs berechnet, um danach relativ zur einem Umfang des Objektes an einer festen Position gehalten zu werden. So wird vorteilhaft eine Berechnungszeit für die Prozessoreinheit reduziert.

Aus den aktuellen Positionen und einer Veränderung dieser Positionen über die Zeit kann neben der Distanz D weiterhin eine Geschwindigkeit der beiden Objekte eines Objektpaares 105 relativ zueinander und eine Richtung R dieser Bewegung als Parameter für den Gefährdungswerte g bestimmt werden. In dem dargestellten Ausführungsbeispiel wertet die Prozessoreinheit 120 zusätzlich zu der Richtung der Bewegung den Anteil r der Bewegung des gefährdenden Objektes in Richtung des zu schützenden Objektes aus. Dies erfolgt durch eine Projektion des entsprechenden Bewegungsvektors der Richtung R auf eine durch die beiden Objekte gebildete Achse A.

In Fig. 3 ist der Anteil r derart ausgebildet, dass er von der Hand 108 in Richtung der Wunde 104 zeigt. Angesichts dieser Bewegung der beiden Objekte des Objektpaares 105 zueinander, wird der aktuelle Gefährdungswerte g größer sein als der in einem früheren Schritt bestimmte Gefährdungswerte, sodass der entsprechend verrechnete Gefährdungsbetrag aktuell größer ist als zu dem früheren Zeitpunkt. Sollte der Gefährdungsbetrag dadurch größer werden als der vorbestimmte Schwellenwert, würde das System 100 ein entsprechendes Alarmierungssignal ausgeben.

In Fig. 4 ist der Anteil r derart ausgebildet, dass er von der Hand 108 in eine von der Wunde 104 wegweisende Richtung zeigt. Angesichts dieser Bewegung der beiden Objekte des Objektpaare 105 weg voneinander, wird der aktuelle Gefährdungswerte G kleiner sein als der in einem früheren Schritt bestimmte Gefährdungswert, sodass der entsprechend verrechnete Gefährdungsbetrag aktuell kleiner ist als zu dem früheren Zeitpunkt. Das Auslösen des Alarmierungssignals wird daher bei der dargestellten Bewegung unwahrscheinlicher als wenn die beiden Objekte an ihrer aktuellen Position verharren würden.

In dem dargestellten Ausführungsbeispiel wird das Verrechnen der Gefährdungswerte dadurch realisiert, dass diese für das zeitliche Auswertungsintervall zu dem Gefährdungsbetrag aufsummiert werden. Hierdurch ergibt sich der Gefährdungsbetrag B rechnerisch gemäß $B\left(t, T, \vec{x}, p_1, ..., p_n\right) = \sum_{i=t-T}^{i=t} g(i, \vec{x}, p_1, ..., p_n)$. Hierbei ist T das zeitliche Auswertungsintervall für das überwachte Objektpaar. T gibt hierbei eine feste Anzahl von diskreten Zeitschritten in die Vergangenheit an, über die der Gefährdungswerte g summiert wird. In einem anderen vorteilhaften Ausführungsbeispiel ergibt sich der Gefährdungsbetrag B rechnerisch gemäß $B\left(t, T, \vec{x}, p_1, ..., p_n\right) = \sum_{i=Z}^{i=t} g(i, \vec{x}, p_1, ..., p_n)$. Hierbei ist Z der Zeitpunkt, ab dem eine Zuordnung zwischen gefährdetem Objekte und zu schützendem Objekt stattgefunden hat und ein entsprechendes Objektpaar zur Überwachung vorliegt.

Bei dem Gefährdungswert g handelt es sich vorzugsweise um eine rationale Zahl, die positiv ist, falls sich das gefährdende Objekt in die Richtung des zu schützenden Objektes bewegt, oder falls sich eine Distanz zwischen diesen beiden Objekten aktuell schneller verkleinert hat als im letzten Zeitschritt, oder falls dem zu schützenden Objekt ein besonders hohes Relevanzniveau zugeordnet ist, oder falls dem gefährdenden Objekt ein besonders hohes Gefahrenniveau zugeordnet ist. Die Abhängigkeit des Gefährdungswertes g von der Distanz kann kontinuierlich, beispielsweise durch eine mit sinkender Distanz monoton steigende Funktion realisiert sein. Der Gefährdungswert g ist null oder negativ, falls sich ein Abstand zwischen dem gefährdenden Objekt und dem zu schützenden Objekt vergrößert, wie dies in Fig. 4 der Fall ist. Weiterhin ist der Gefährdungswerte null oder negativ, falls ein Gegenstand den direkten Weg zwischen gefährdendem Objekt und zu schützendem Objekt versperrt, wie dies in Fig. 5 durch einen zwischen der Hand 108 und der Wunde 104 befindlichen Gegenstand 570, nämlich ein Tablett, der Fall ist. Ein negativer oder bei null stagnierender Gefährdungswert sorgt dafür, dass der Gefährdungsbetrag nicht größer wird, da insbesondere beim Verrechnen durch Aufsummieren gemäß dem vorliegenden Ausführungsbeispiel keine positive Zahl auf den vorhergehenden Gefährdungsbetrag addiert wird.

In einem konkreten Berechnungsbeispiel für die in Fig. 3 dargestellte Gefährdungssituation wurde nach einer anfänglichen Bewegung der Hand 108 in Richtung der Wunde 104 ein Gefährdungswert von 0 auf 1 verändert, sodass der Gefährdungsbetrag auch 1 betrug. Nachdem die Hand 108 dann für einige Zeit in einer Position verharrte, wurde der Gefährdungswert wieder auf 0 verändert und der Gefährdungsbetrag blieb bei 1. Mit der dargestellten erneut einsetzenden Bewegung wurde der Gefährdungswert auf 2 verändert, da nun neben der Bewegung auch die Distanz unterhalb eines entsprechenden Schwellenwertes ist, sodass hierdurch der Gefährdungswert zusätzlich um 1 erhöht wird, entsprechend der ab dem Schwellenwert 0,5 m einsetzenden inversen Abhängigkeit $g_D = 0{,}5/D$ mit der bestimmten Distanz D=0,5 m. Hierdurch liegt der Gefährdungsbetrag nun bei 3. Vorliegend wird ab dem Schwellenwert des Gefährdungsbetrags von 4,5 das Alarmierungssignal ausgegeben. Daher wird bei einem erneuten Zeitschritt, in dem die Hand sich auf die Wunde zu bewegt dieses Alarmierungssignal ausgelöst werden, falls nicht vorher eine Bewegung von der Wunde weg erfolgt, was zu einem negativen Gefährdungswert und einer entsprechenden Reduzierung des Gefährdungsbetrags führen würde.

Vorzugsweise ist der Gefährdungswert linear abhängig von dem Wichtigkeitswert, der dem jeweiligen Objektpaar durch die Prozessoreinheit zugeordnet ist. Der Wichtigkeitswert ist vorzugsweise proportional abhängig von dem vorbestimmten Relevanzniveau des zu schützenden Objekts und/oder dem vorbestimmten Gefahrenniveau des gefährdenden Objekts.

Das Festlegen konkreter rationaler Zahlen für das Gefahrenniveau und/oder das Relevanzniveau und/oder den Wichtigkeitswert und/oder den Gefährdungswert erfolgt in einem bevorzugten Ausführungsbeispiel durch eine manuelle Nutzereingabe und/oder durch eine vom Hersteller bereitgestellte vorbestimmte Zuordnung von Werten, die in einem Speicher der Prozessoreinheit gespeichert ist. Hierdurch können individuelle Präferenzen bei der Beurteilung einer Gefährdungssituation berücksichtigt werden.

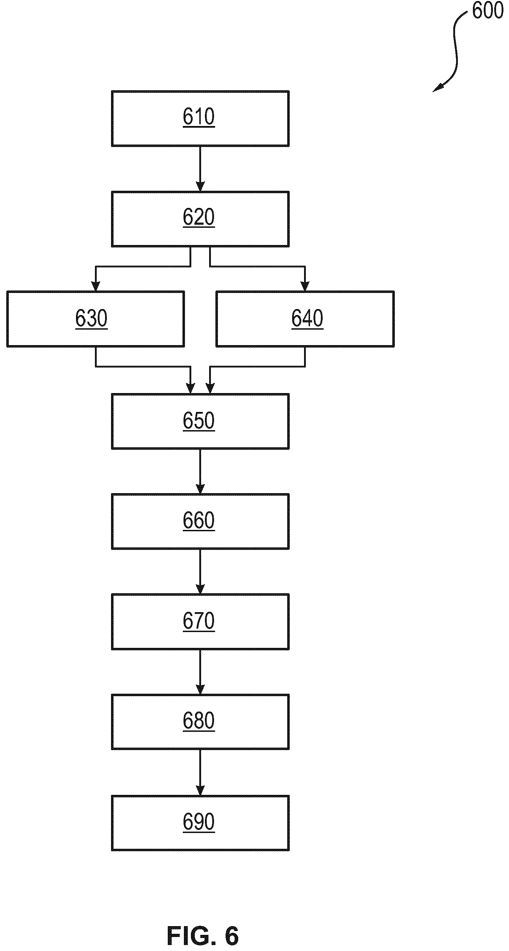

Fig. 6 zeigt ein Flussdiagramm eines ersten Ausführungsbeispiels eines erfindungsgemäßen Verfahrens 600.

Das Verfahren 600 zur automatischen Erkennung von Gefährdungssituationen innerhalb eines Überwachungsbereichs für Objekte im medizinischen Umfeld, weist eine Abfolge der im Folgenden beschriebenen Schritte auf:

Ein erster Schritt 610 umfasst ein Bestimmen eines Sensorsignals, das Sensordaten umfasst, die eine Abfolge von dreidimensionalen Darstellungen des Überwachungsbereichs über eine Überwachungsdauer indizieren, und ein Ausgeben des Sensorsignals in Echtzeit.

Ein darauffolgender Schritt 620 umfasst ein Empfangen des Sensorsignals.

Ein nächster Schritt 630 umfasst ein Festlegen einer Anzahl zu schützender Objekte innerhalb des Überwachungsbereichs anhand der indizierten dreidimensionalen Darstellungen.

Ein zum Schritt 630 parallel ausführbarer Schritt 640 umfasst ein Erkennen, insbesondere ein automatisiertes Erkennen von gefährdenden Objekten innerhalb des Überwachungsbereichs anhand der indizierten dreidimensionalen Darstellungen.

Ein weiterer Schritt 650 umfasst ein Bestimmen einer Anzahl von Objektpaaren durch eine Zuordnung zwischen einem zu schützenden Objekt aus der Anzahl zu schützender Objekte und einem gefährdenden Objekten.

Ein darauffolgender Schritt 660 umfasst ein Zuordnen eines aktuellen Gefährdungswertes zu einem jeweiligen Objektpaar abhängig von den aktuellen Positionen des zu schützenden Objekts und des gefährdenden Objekts und von einer Gruppe von sich aus einer Auswertung früherer Auswertedaten ergebender Parameter.

Ein nächster Schritt 670 umfasst ein Verrechnen der aktuellen Gefährdungswerte für ein jeweiliges Objektpaar zu einem jeweiligen Gefährdungsbetrag über ein zeitliches Auswertungsintervall bis zum aktuellen Zeitpunkt.

Ein weiterer Schritt 680 umfasst ein Ausgeben eines entsprechenden Alarmierungssignals, falls der so bestimmte Gefährdungsbetrag einen vorbestimmten Schwellenwert übersteigt.

Ein abschließender Schritt 690 umfasst ein Empfangen des Alarmierungssignals und ein Auslösen eines Alarms abhängig von dem Alarmierungssignal.

Die Schritte 610 und 620 werden typischerweise in Echtzeit, also in kurz aufeinanderfolgenden Zeitschritten wiederholt ausgeführt.

Die Schritte 630 und 640 können parallel zueinander oder in einer beliebigen Reihenfolge aufeinanderfolgend ausgeführt werden.

Der Schritt 650 wird während der Überwachung eines Objektpaares einmal ausgeführt, wohingegen die Schritte 660 und 670 den eigentlichen Überwachungsvorgang beschreiben und in aufeinanderfolgenden Zeitschritten wiederholt ausgeführt werden, sodass der jeweilige Gefährdungsbetrag immer mit einem der aktuellen Situation in dem Überwachungsbereich angepassten Gefährdungswert verrechnet wird. Die Schritte 680 und 690 werden im Rahmen der Überwachung eines Objektpaares vorzugsweise nur einmal nacheinander ausgeführt, nämlich nach dem Übersteigen des vorbestimmten Schwellenwertes durch den Gefährdungsbetrag.

**In** einem bevorzugten Ausführungsbeispiel erfolgt das Verrechnen der aktuellen Gefährdungswerte entsprechend Schritt 670 durch ein Aufsummieren dieser Gefährdungswerte über das zeitliche Auswertungsintervall bis zum aktuellen Zeitpunkt.

Vorzugsweise umfasst das erfindungsgemäße Verfahren in einem weiteren Schritt ein Deaktivieren des Verfahrens 600, falls die Prozessoreinheit durch das Sensorsignal die Anwesenheit von medizinischem Personal innerhalb des Überwachungsbereichs erfasst. In einem bevorzugten Beispiel dieser Variante wird das Verfahren 600 mit den vor dem Deaktivieren des medizinischen Personals erfassten Daten weitergeführt, wenn das erfasste medizinische Personal den Überwachungsbereich verlassen hat. In einer Variante dieser Ausführungsform wird das medizinische Personal von anderen Personen durch ein automatisches Erfassen einer von dem medizinischen Personal mitgeführten Identifikationsnummer realisiert. Dies ist beispielsweise durch eine mitgeführte Schlüsselkarte, auf der die Identifikationsnummer hinterlegt ist, und/oder durch eine entsprechende Anordnung einer Identifikationsinformation auf der Kleidung des medizinischen Personals möglich. In einem alternativen oder ergänzenden Beispiel wird die Identifikationsinformation manuell durch das medizinische Personal bereitgestellt.

## **Bezugszeichenliste**

| | |
|---|---|
| 100, 200 | System |
| 102 | Überwachungsbereich |
| 103, 103' | Teilüberwachungsbereiche |
| 104 | Wunde |
| 105 | Objektpaar |
| 106 | Patient |
| 108 | Hand |
| 109 | Kopf |
| 110, 210 | Sensoreinheit |
| 112, 212, 212' | Anzahl von optischen Sensoren |
| 114 | Sensorsignal |
| 120 | Prozessoreinheit |
| 121 | erster Verarbeitungsschritt |
| 122 | zu schützendes Objekt |
| 123 | zweiter Verarbeitungsschritt |
| 124 | gefährdendes Objekt |
| 125 | Zuordnungsschritt |
| 126 | Alarmierungssignal |
| 127 | Überwachungsvorgang |
| 130, 230 | Ausgabeeinheit |
| 132 | Alarm |
| 134 | Lautsprecher |
| 236 | Auslösesignal |
| 240 | Gehäuse |
| 250 | Benutzerschnittstelle |
| 252 | Touchdisplay |
| 254 | Benutzereingabe |
| 256 | Eingabesignal |
| 260 | Kommunikationsnetzwerk |
| 570 | weiterer Gegenstand |
| 600 | Verfahren |
| 610, 620, 630, 640, 650, 660, 670, 680, 690 | Verfahrensschritte |
| D | Abstand zwischen überwachten Objekten |
| R | Bewegungsrichtung |
| r | Anteil der Bewegung |

A                                                    Achse zwischen überwachten Objekten

**Patentansprüche**

1.  System (100) zur automatischen Erkennung von Gefährdungssituationen innerhalb eines Überwachungsbereichs (102) für Objekte im medizinischen Umfeld, mit

    - einer Sensoreinheit (110) mit einer Anzahl von optischen Sensoren (112), die ausgebildet ist, ein Sensorsignal (114) zu bestimmen und in Echtzeit auszugeben,
    wobei die Anzahl von optischen Sensoren (112) weiterhin derart in dem medizinischen Umfeld anordenbar und derart ausgebildet ist, dass das Sensorsignal (114) Sensordaten umfasst, die eine Abfolge von dreidimensionalen Darstellungen des Überwachungsbereichs (102) über eine Überwachungsdauer indizieren,
    - einer Prozessoreinheit (120), die ausgebildet ist,

    - das Sensorsignal (114) zu empfangen,
    - anhand der indizierten dreidimensionalen Darstellungen eine Festlegung einer Anzahl zu schützender Objekte (122) innerhalb des Überwachungsbereichs (102) auszuführen,
    wobei die Anzahl zu schützender Objekte (122) mindestens ein Objekt ist aus der Gruppe: Infusion, Medizingerät, Kabel, Schlauch, Tubus, Verband, Wunde (104),
    - anhand der indizierten dreidimensionalen Darstellungen eine Erkennung von gefährdenden Objekten (124) innerhalb des Überwachungsbereichs (102) auszuführen,
    wobei die gefährdenden Objekte (124) mindestens ein Objekt aus der Gruppe: Hand (108) eines Patienten (106), Fuß eines Patienten (106), Kopf (109) eines Patienten (106), Tier, Bettgitter sind,
    - eine Anzahl von Objektpaaren (105) durch eine Zuordnung zwischen einem zu schützenden Objekt (122) aus der Anzahl zu schützender Objekte (122) und einem gefährdenden Objekt (124) zu bestimmen,
    - im Laufe der Überwachungsdauer die Positionen von zu schützendem Objekt (122) und gefährdendem Objekt (124) eines jeweiligen Objektpaares (105) innerhalb des Überwachungsbereichs (102) zu überwachen,
    - abhängig von den beiden aktuellen Positionen und einer Gruppe von sich aus einer Auswertung früherer Auswertedaten ergebender Parameter einem jeweiligen Objektpaar (105) einen aktuellen Gefährdungswert zuzuordnen,
    wobei die sich ergebenden Parameter zumindest eine Distanz (D) zwischen den Objekten eines Objektpaares (105) und eine Richtung (R) einer Bewegung der Objekte eines Objektpaares (105) relativ zueinander sind, und
    - über ein zeitliches Auswertungsintervall für jedes Objektpaar (105) jeweils die aktuellen Gefährdungswerte bis zum aktuellen Zeitpunkt aufzusummieren und ein entsprechendes Alarmierungssignal (126) auszugeben, falls ein so verrechneter Gefährdungsbetrag eines Objektpaares (105) einen vorbestimmten Schwellenwert übersteigt,

    - eine Ausgabeeinheit (130), die ausgebildet ist, das Alarmierungssignal (126) zu empfangen und abhängig von dem Alarmierungssignal (126) einen Alarm (132) auszulösen.

2.  System (100) gemäß Anspruch 1,
    wobei die Zuordnung zwischen einem zu schützenden Objekt (122) und einem gefährdenden Objekt (124) abhängig ist von einer aktuellen Position des gefährdenden Objekts (124) innerhalb des Überwachungsbereichs (102) relativ zu dem zu schützenden Objekt (122).

3.  System (100) gemäß mindestens einem der vorhergehenden Ansprüche, wobei jedem Objektpaar (105) durch die Prozessoreinheit (120) ein Wichtigkeitswert zugeordnet wird, der sich aus einem vorbestimmten Relevanzniveau des zu schützenden Objekts (122) und/oder aus einem vorbestimmten Gefahrenniveau des gefährdenden Objekts (124) ergibt, und wobei der aktuelle Gefährdungswert des Objektpaares (105) abhängig von dem Wichtigkeitswert ist.

4.  System (100) gemäß mindestens einem der vorhergehenden Ansprüche, wobei der aktuelle Gefährdungswert eines Objektpaares (105) auch abhängig davon ist, ob eine direkte Wegstrecke zwischen dem zu schützenden Objekt (122) und dem gefährdenden Objekt (124) durch einen weiteren Gegenstand (570) versperrt ist.

5.  System (100) gemäß mindestens einem der vorhergehenden Ansprüche, wobei die Festlegung der Anzahl zu schützender Objekte (122) und/oder die Erkennung von gefährdenden Objekten durch eine automatisierte Objekterkennung realisiert ist.

6. System (100) gemäß mindestens einem der vorhergehenden Ansprüche, wobei die Festlegung der Anzahl zu schützender Objekte (122) zumindest teilweise durch eine Interaktion mit einem Nutzer des Systems (100) realisiert ist.

7. System (100) gemäß mindestens einem der vorhergehenden Ansprüche, wobei das zeitliche Auswertungsintervall dasjenige Zeitintervall ist, das durch einen Bestimmungszeitpunkt für die Bestimmung des entsprechenden Objektpaares (105) und dem aktuellen Zeitpunkt gebildet ist.

8. System (200) gemäß mindestens einem der vorhergehenden Ansprüche, weiterhin aufweisend eine Benutzerschnittstelle (250), die mit der Prozessoreinheit (120) verbunden ist, und die ausgebildet ist, eine manuelle Steuerung der Festlegung eines zu schützenden Objekts (122), der Erkennung eines gefährdenden Objekts (124) und/oder der Zuordnung der beiden Objekte zu einem Objektpaar (105) zu ermöglichen.

9. System (200) gemäß mindestens einem der vorhergehenden Ansprüche, wobei das Alarmierungssignal (126) das zu schützende Objekt (122) und/oder das gefährdende Objekt (124) indiziert.

10. Verfahren (600) zur automatischen Erkennung von Gefährdungssituationen innerhalb eines Überwachungsbereichs (102) für Objekte im medizinischen Umfeld, aufweisend die Schritte

- Bestimmen eines Sensorsignals (114), das Sensordaten umfasst, die eine Abfolge von dreidimensionalen Darstellungen des Überwachungsbereichs (102) über eine Überwachungsdauer indizieren,
- Ausgeben des Sensorsignals (114) in Echtzeit,
- Empfangen des Sensorsignals (114),
- Festlegen einer Anzahl zu schützender Objekte (122) innerhalb des Überwachungsbereichs (102) anhand der indizierten dreidimensionalen Darstellungen,
wobei die Anzahl zu schützender Objekte (122) mindestens ein Objekt ist aus der Gruppe: Infusion, Medizingerät, Kabel, Schlauch, Tubus, Verband, Wunde (104),
- Erkennen von gefährdenden Objekten (124) innerhalb des Überwachungsbereichs (102) anhand der indizierten dreidimensionalen Darstellungen,
wobei die gefährdenden Objekte (124) mindestens ein Objekt aus der Gruppe: Hand (108) eines Patienten (106), Fuß eines Patienten (106), Kopf (109) eines Patienten (106), Tier, Bettgitter sind,
- Bestimmen einer Anzahl von Objektpaaren (105) durch eine Zuordnung zwischen einem zu schützenden Objekt aus der Anzahl zu schützender Objekte (122) und einem gefährdenden Objekt (124),
- Zuordnen eines aktuellen Gefährdungswertes zu einem jeweiligen Objektpaar (105) abhängig von den aktuellen Positionen des zu schützenden Objekts (122) und des gefährdenden Objekts (124) und von einer Gruppe von sich aus einer Auswertung früherer Auswertedaten ergebender Parameter,
wobei die sich ergebenden Parameter zumindest eine Distanz (D) zwischen den Objekten eines Objektpaares (105) und eine Richtung (R) einer Bewegung der Objekte eines Objektpaares (105) relativ zueinander sind,
- Verrechnen der aktuellen Gefährdungswerte für ein jeweiliges Objektpaar (105) zu einem jeweiligen Gefährdungsbetrag über ein zeitliches Auswertungsintervall bis zum aktuellen Zeitpunkt,
wobei das Verrechnen der aktuellen Gefährdungswerte ein Aufsummieren dieser aktuellen Gefährdungswerte bis zum aktuellen Zeitpunkt zu dem jeweiligen Gefährdungsbetrag des Objektpaares (105) umfasst,
- Ausgeben eines entsprechenden Alarmierungssignals (126), falls der so bestimmte Gefährdungsbetrag einen vorbestimmten Schwellenwert übersteigt, und
- Empfangen des Alarmierungssignals (126) und Auslösen eines Alarms (132) abhängig von dem Alarmierungssignal (126).

**Claims**

1. System (100) for automatically detecting hazardous situations within a monitoring region (102) for objects in a medical environment, comprising

- a sensor unit (110) having a number of optical sensors (112), which is designed to determine a sensor signal (114) and output it in real time,
wherein the number of optical sensors (112) can also be arranged in the medical environment and designed such that the sensor signal (114) comprises sensor data which indicate a sequence of three-dimensional representations of the monitoring region (102) over a monitoring period,
- a processor unit (120) which is designed
- to receive the sensor signal (114),
- to establish a number of objects (122) to be protected within the monitoring region (102) on the basis of the indicated three-dimensional representations,
wherein the number of objects (122) to be protected is at least one object from the group: infusion, medical device, cable, hose, tube, bandage, wound (104),
- to detect hazardous objects (124) within the monitoring region (102) on the basis of the indicated three-dimensional representations,
wherein the hazardous objects (124) are at least one object from the group: hand (108) of a patient (106), foot of a patient (106), head (109) of a patient (106), animal, bed rail,
- to determine a number of object pairs (105) by an assignment between an object (122) to be protected from the number of objects (122) to be protected and a hazardous object (124),
- to monitor the positions of the object (122) to be protected and the hazardous object (124) of a relevant object pair (105) within the monitoring region (102) during the monitoring period,
- to assign a current hazard value to a relevant object pair (105) depending on the two current positions and a group of parameters resulting from an evaluation of previous evaluation data,
wherein the resulting parameters are at least a distance (D) between the objects of an object pair (105) and a direction (R) of movement of the objects of an object pair (105) relative to one another, and
- to sum up each of the current hazard values up to the current point in time for each object pair (105) over a temporal evaluation interval, and to issue a corresponding alarm signal (126) if a thus calculated hazard amount of an object pair (105) exceeds a predetermined threshold value,
- an output unit (130) which is designed to receive the alarm signal (126) and to trigger an alarm (132) depending on the alarm signal (126).

2. System (100) according to claim 1,
wherein the assignment between an object (122) to be protected and a hazardous object (124) depends on a current position of the hazardous object (124) within the monitoring region (102) relative to the object (122) to be protected.

3. System (100) according to at least one of the preceding claims,
wherein the processor unit (120) assigns each object pair (105) an importance value which results from a predetermined relevance level of the object (122) to be protected and/or from a predetermined hazard level of the hazardous object (124), and wherein the current hazard value of the object pair (105) depends on the importance value.

4. System (100) according to at least one of the preceding claims,
wherein the current hazard value of an object pair (105) also depends on whether a direct route between the object (122) to be protected and the hazardous object (124) is obstructed by another item (570).

5. System (100) according to at least one of the preceding claims,
wherein the establishment of the number of objects (122) to be protected and/or the detection of hazardous objects is achieved by means of automated object detection.

6. System (100) according to at least one of the preceding claims,
wherein the establishment of the number of objects (122) to be protected is at least partially achieved by means of an interaction with a user of the system (100).

7. System (100) according to at least one of the preceding claims,
wherein the temporal evaluation interval is the time interval which is formed by a determination time for the determination of the corresponding object pair (105) and the current time.

8. System (200) according to at least one of the preceding claims,
further comprising a user interface (250) which is connected to the processor unit (120) and which is designed to allow manual control of establishing an object (122) to be protected, detecting a hazardous object (124) and/or assigning the two objects to an object pair (105).

9. System (200) according to at least one of the preceding claims,

wherein the alarm signal (126) indicates the object (122) to be protected and/or the hazardous object (124).

10. Method (600) for automatically detecting hazardous situations within a monitoring region (102) for objects in a medical environment, comprising the steps of

- determining a sensor signal (114) comprising sensor data which indicate a sequence of three-dimensional representations of the monitoring region (102) over a monitoring period,
- outputting the sensor signal (114) in real time,
- receiving the sensor signal (114),
- establishing a number of objects (122) to be protected within the monitoring region (102) on the basis of the indicated three-dimensional representations,
wherein the number of objects (122) to be protected is at least one object from the group: infusion, medical device, cable, hose, tube, bandage, wound (104),
- detecting hazardous objects (124) within the monitoring region (102) on the basis of the indicated three-dimensional representations,
wherein the hazardous objects (124) are at least one object from the group: hand (108) of a patient (106), foot of a patient (106), head (109) of a patient (106), animal, bed rail,
- determining a number of object pairs (105) by an assignment between an object to be protected from the number of objects (122) to be protected and a hazardous object (124),
- assigning a current hazard value to a relevant object pair (105) depending on the current positions of the object (122) to be protected and the hazardous object (124) and depending on a group of parameters resulting from an evaluation of previous evaluation data,
wherein the resulting parameters are at least a distance (D) between the objects of an object pair (105) and a direction (R) of movement of the objects of an object pair (105) relative to one another,
- performing a calculation on the current hazard values for a relevant object pair (105) to obtain a relevant hazard amount over a temporal evaluation interval up to the current time,
wherein performing a calculation on the current hazard values comprises summing up these current hazard values up to the current point in time to obtain the relevant hazard amount of the object pair (105),
- outputting a corresponding alarm signal (126) if the thus determined hazard amount exceeds a predetermined threshold value, and
- receiving the alarm signal (126) and triggering an alarm (132) depending on the alarm signal (126).

**Revendications**

1. Système (100) pour la reconnaissance automatique de situations dangereuses à l'intérieur d'une zone de surveillance (102) pour des objets dans un environnement médical, comportant

- une unité à capteurs (110) comportant un certain nombre de capteurs optiques (112), laquelle est configurée pour déterminer un signal de capteur (114) et l'émettre en temps réel,
dans lequel le nombre de capteurs optiques (112) peut en outre être disposé dans l'environnement médical et est configuré de telle sorte que le signal de capteur (114) comprend des données de capteur indiquant une séquence de représentations tridimensionnelles de la zone de surveillance (102) sur une période de surveillance,
- une unité de traitement (120) configurée pour
- recevoir le signal de capteur (114),
- effectuer une définition d'un certain nombre d'objets à protéger (122) à l'intérieur de la zone de surveillance (102) à l'aide des représentations tridimensionnelles indiquées,
dans lequel le nombre d'objets à protéger (122) est au moins un objet du groupe : perfusion, appareil médical, câble, tuyau, tube, bandage, plaie (104),
- effectuer une reconnaissance d'objets dangereux (124) à l'intérieur de la zone de surveillance (102) à l'aide des représentations tridimensionnelles indiquées,
dans lequel les objets dangereux (124) sont au moins un objet du groupe : main (108) d'un patient (106), pied d'un patient (106), tête (109) d'un patient (106), animal, barrières de lit,
- déterminer un certain nombre de paires d'objets (105) par une association entre un objet à protéger (122) parmi le nombre d'objets à protéger (122) et un objet dangereux (124),
- surveiller, au cours de la période de surveillance, les positions de l'objet à protéger (122) et de l'objet dangereux (124) d'une paire d'objets (105) respective à l'intérieur de la zone de surveillance (102),
- associer une valeur de danger actuelle à une paire d'objets (105) respective en fonction des deux positions actuelles et d'un groupe de paramètres résultant d'une évaluation de données d'évaluation antérieures, dans lequel les paramètres résultants sont au moins une distance (D) entre les objets d'une paire d'objets (105) et une direction (R) d'un déplacement des objets d'une paire d'objets (105) l'un par rapport à l'autre, et

- additionner, sur un intervalle d'évaluation temporel, pour chaque paire d'objets (105), respectivement les valeurs de danger actuelles jusqu'à l'instant actuel et émettre un signal d'alarme (126) correspondant si une grandeur de danger ainsi calculée d'une paire d'objets (105) franchit une valeur seuil prédéterminée,
- une unité d'émission (130) configurée pour recevoir le signal d'alarme (126) et pour déclencher une alarme (132) en fonction du signal d'alarme (126).

2.  Système (100) selon la revendication 1,
    dans lequel l'association entre un objet à protéger (122) et un objet dangereux (124) dépend d'une position actuelle de l'objet dangereux (124) à l'intérieur de la zone de surveillance (102) par rapport à l'objet à protéger (122).

3.  Système (100) selon au moins l'une des revendications précédentes,
    dans lequel une valeur d'importance est associée à chaque paire d'objets (105) par l'unité de traitement (120), laquelle valeur d'importance résulte d'un niveau de pertinence prédéterminé de l'objet à protéger (122) et/ou d'un niveau de danger prédéterminé de l'objet dangereux (124), et dans lequel la valeur de danger actuelle de la paire d'objets (105) dépend de la valeur d'importance.

4.  Système (100) selon au moins l'une des revendications précédentes,
    dans lequel la valeur de danger actuelle d'une paire d'objets (105) dépend également du fait de savoir si un parcours direct entre l'objet à protéger (122) et l'objet dangereux (124) est, ou non, obstrué par un autre produit (570).

5.  Système (100) selon au moins l'une des revendications précédentes,
    dans lequel la définition du nombre d'objets à protéger (122) et/ou la reconnaissance d'objets dangereux sont réalisées par une reconnaissance d'objets automatisée.

6.  Système (100) selon au moins l'une des revendications précédentes,
    dans lequel la définition du nombre d'objets à protéger (122) est réalisée au moins partiellement par une interaction avec un utilisateur du système (100).

7.  Système (100) selon au moins l'une des revendications précédentes,
    dans lequel l'intervalle d'évaluation temporel est l'intervalle de temps formé par un instant de détermination pour la détermination de la paire d'objets (105) correspondante et l'instant actuel.

8.  Système (200) selon au moins l'une des revendications précédentes,
    présentant en outre une interface utilisateur (250) connectée à l'unité de traitement (120) et configurée pour permettre une commande manuelle de la définition d'un objet à protéger (122), de la reconnaissance d'un objet dangereux (124) et/ou de l'association des deux objets à une paire d'objets (105).

9.  Système (200) selon au moins l'une des revendications précédentes,
    dans lequel le signal d'alarme (126) indique l'objet à protéger (122) et/ou l'objet dangereux (124).

10. Procédé (600) pour la reconnaissance automatique de situations dangereuses à l'intérieur d'une zone de surveillance (102) pour des objets dans un environnement médical, présentant les étapes consistant à

    - déterminer un signal de capteur (114) comprenant des données de capteur indiquant une séquence de représentations tridimensionnelles de la zone de surveillance (102) sur une période de surveillance,
    - émettre le signal de capteur (114) en temps réel,
    - recevoir le signal de capteur (114),
    - définir un certain nombre d'objets à protéger (122) à l'intérieur de la zone de surveillance (102) à l'aide des représentations tridimensionnelles indiquées,
    dans lequel le nombre d'objets à protéger (122) est au moins un objet du groupe : perfusion, appareil médical, câble, tuyau, tube, bandage, plaie (104),
    - reconnaître des objets dangereux (124) à l'intérieur de la zone de surveillance (102) à l'aide des représentations tridimensionnelles indiquées,
    dans lequel les objets dangereux (124) sont au moins un objet du groupe : main (108) d'un patient (106), pied d'un patient (106), tête (109) d'un patient (106), animal, barrières de lit,

- déterminer un certain nombre de paires d'objets (105) par une association entre un objet à protéger parmi le nombre d'objets à protéger (122) et un objet dangereux (124),
- associer une valeur de danger actuelle à une paire d'objets (105) respective en fonction des positions actuelles de l'objet à protéger (122) et de l'objet dangereux (124) et d'un groupe de paramètres résultant d'une évaluation de données d'évaluation antérieures,
dans lequel les paramètres résultants sont au moins une distance (D) entre les objets d'une paire d'objets (105) et une direction (R) d'un déplacement des objets d'une paire d'objets (105) l'un par rapport à l'autre,
- calculer les valeurs de danger actuelles pour une paire d'objets (105) respective en une grandeur de danger respective sur un intervalle d'évaluation temporel jusqu'à l'instant actuel,
dans lequel le calcul des valeurs de danger actuelles comprend une somme desdites valeurs de danger actuelles jusqu'à l'instant actuel pour obtenir la grandeur de danger respective de la paire d'objets (105),
- émettre un signal d'alarme (126) correspondant si la grandeur de danger ainsi déterminée franchit un seuil prédéterminé, et
- recevoir le signal d'alarme (126) et déclencher une alarme (132) en fonction du signal d'alarme (126).

**FIG. 1**

EP 3 748 536 B1

FIG. 2

FIG. 4

FIG. 3

**FIG. 5**

EP 3 748 536 B1

600

610

620

630    640

650

660

670

680

690

FIG. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 10090068 B
- CN 106233349 A
- US 10043360 B1
- WO 2019011769 A1